# EUROPEAN PATENT APPLICATION

(11) **EP 4 344 796 A1**
(43) Date of publication of application: **03.04.2024**
(21) Application number: 22198066.7
(22) Date of filing: 27.09.2022
(51) Int. Cl.: B06B 1/06, A61B 8/00

(54) **METHOD OF MANUFACTURING A CURVILINEAR ULTRASOUND TRANSDUCER FOR USE WITH AN ENDOSCOPE**

(71) Applicant: Ambu A/S, 2750 Ballerup (DK)
(72) Inventor: DIEDERICHSEN, Søren Elmin, 2750 Ballerup (DK); SONNENBORG, Finn, 3600 Frederikssund (DK); SØRENSEN, Morten, 2750 Ballerup (DK)
(74) Representative: AWA Denmark A/S

(57) **Abstract**

Disclosed is a method of manufacturing a curvilinear ultrasound transducer comprising, providing a piezoelectric block, providing a first acoustic matching layer, cutting the piezoelectric block at a plurality of predetermined positions forming a plurality of ultrasound transducer elements separated by a plurality of gaps, allowing the plurality of gaps to be filed with a first gas resulting in a plurality of gas-filled gaps, arranging the first acoustic matching layer above the piezoelectric block, optionally after the piezoelectric block has been cut, providing a support structure having a curvilinear upper surface, arranging the piezoelectric block with the first acoustic matching layer above the curvilinear upper surface of the support structure, optionally after the piezoelectric block has been cut, providing a pressure element having a concave surface, and moving the support structure and the pressure element together to shape the piezoelectric block and first acoustic matching layer curvilinear.

## Description

### Field

The present disclosure relates to a method of manufacturing a curvilinear ultrasound transducer, a curvilinear ultrasound transducer, and an endoscope.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side.

The image capturing device is however restricted by the limited penetration depth of visible light.

To examining deeper structures other imaging modalities must be used.

One possibility is to provide the endoscope with a curvilinear ultrasound probe. By using emission of ultrasound waves deeper structures may be examined.

Curvilinear ultrasound probes are however complex to manufacture.

Ultrasound endoscopes are therefore significantly more expensive than regular endoscopes.

Availability of endoscopic ultrasound is therefore limited for some patient groups.

Furthermore, endoscopes are difficult to clean and there exist the risk of cross-contamination, i.e. that a pathological condition is transferred between patients as result of improper cleaning.

The most secure solution has been the introduction of single-use endoscopes. A single use endoscope eliminates any risk of cross-contamination.

The cost of ultrasound probes has however made it difficult to provide single use ultrasound endoscopes. This is problematic since the provision of an ultrasound probe at the distal end of the endoscope makes the endoscope even harder to clean.

Thus, it remains a problem to provide an improved method of manufacturing an ultrasound probe and / or providing an improved ultrasound probe.

### Summary

According to a first aspect, the present disclosure relates to a method of manufacturing a curvilinear ultrasound transducer for use with an endoscope, the method comprising,
providing a piezoelectric block having an upper side and a lower side opposite to the upper side,
providing a first acoustic matching layer,
cutting the piezoelectric block at a plurality of positions forming a plurality of ultrasound transducer elements separated by a plurality of gaps,
allowing the plurality of gaps to be filed with a first gas resulting in a plurality of gas-filled gaps,
arranging the first acoustic matching layer above the piezoelectric block, optionally after the piezoelectric block has been cut,
providing a support structure having a curvilinear upper surface,
arranging the piezoelectric block with the first acoustic matching layer above the curvilinear upper surface of the support structure, optionally after the piezoelectric block has been cut,
providing a pressure element, and
moving the support structure and the pressure element together to shape the piezoelectric block and first acoustic matching layer curvilinear.

Consequently, by using a first gas to fill the plurality of gaps between the transducer elements instead of a traditional kerf filler material, the transducer elements may be curvilinear shaped together with the first acoustic matching layer. This may simplify the process of manufacturing a curvilinear ultrasound transducer Specifically, the number of process steps may be reduced. Furthermore, it may become simpler to produce the first acoustic matching layer and potential additional layers. It may also become simpler to arrange the first acoustic matching layer and potential additional layers on the piezoelectric block.

The piezoelectric block may be made of any kind of piezoelectric material such as lead zirconate titanate (PZT), Quartz, Barium titanate, Poly(vinylidene fluoride) (PVDF), or a piezoelectric composite material. The upper surface of the piezoelectric block may extend in a first reference plane and the lower surface of the piezoelectric block may extend in a second reference plane, before the piezo electric block has been curvilinear shaped. The first reference plane and the second reference plane may be parallel.

The first acoustic matching layer is configured to provide an acoustic impedance gradient between the acoustic impedance of the tissue and the acoustic impedance of the ultrasound transducer elements. The first acoustic matching layer may be made of epoxy, polyurethane, or polystyrene. Additional acoustic matching layers may be provided e.g. a second acoustic matching layer and a third acoustic matching layer. Potential additional acoustic matching layers are preferably also arranged above the piezo electric block before the piezo electric block has been curvilinear shaped.

The pressure in the gas-filed gaps at room temperature (20 degrees Celsius) may be lower than 1 Atmosphere (101326 Pa) e.g. the pressure may be below 0.8 Atmosphere, 0.5 Atmosphere, or 0.1 Atmosphere at room temperature (20 degrees Celsius).

This may limit the transfer of acoustic energy between the transducer elements.

The positions where the piezoelectric block is may be predetermined.

The pressure element may be a concave surface. The concave surface may be formed by a single curved surface or a plurality of piecewise flat surfaces together forming the concave surface. Alternatively, the pressure element may be a roller pressure element starting from one end and applying pressure while rolling over to the other end.

The gas filled gaps may be sealed to prevent the pressure from increasing. The first acoustic matching layers, the support structure or other elements of the ultrasound transducer may assist in sealing the gas filed gaps. Additionally / alternatively, the piezoelectric block may be encapsulated e.g. in an epoxy resin or the like.

The support structure and the pressure element may be moved together by having the support structure being kept stationary e.g. support by an external support, and moving the pressure element toward the support structure. Alternatively, the pressure element may be kept stationary and the support structure may be moved towards the pressure element. Alternatively, the support structure may be moved towards the pressure element and the pressure element may be moved towards the support structure. The pressure element may apply a pressure between 0.5 bar and 5 bar or between 0.8 bar and 2.5 bar. As an example, the pressure may be 1 bar. The piezoelectric block may be heated before, while and / or after the support structure and the pressure element are moved together. The piezoelectric block may be heated to a temperature between 40 degrees and 120 degrees or 60 and 100 degrees Celsius before the support structure and the pressure element are moved together. The piezoelectric block may be heated to a temperature between 25 degrees and 90 degrees or 30 and 50 degrees Celsius after the support structure and the pressure element have been moved together. As an example, the piezoelectric block may be heated to a temperature of 80 degrees for one hour, then a uniaxial pressure of 1 bar is applied using the pressure element, and finally the piezoelectric block is cured for 12 hours at 40 degrees Celsius.

The piezoelectric block is preferably permanently attached to the support structure. An adhesive may be applied between the piezoelectric block and the curvilinear upper surface of the support structure. As an example, the adhesive may be a two-component epoxy glue. The piezo electric block is preferably permanently attached to the support structure when the support structure and the pressure element is moved together.

The support structure is preferably made of a material configured to absorb ultrasound energy. The material may be a composite material. As an example, the material may be a epoxy mixed with tungsten powder.

The first gas may consist of a single type of gas or a combination of different types of gases. The first gas preferably stays in the gaseous phase at least within a normal temperature range such as from -10 degrees Celsius to +90 degrees Celsius.

In some embodiments, the first gas is atmospheric air.

In some embodiments, the first acoustic matching layer is arranged above the plurality of ultrasound transducer elements after the piezoelectric block has been cut forming the plurality of ultrasound transducer elements.

Consequently, the first acoustic matching layer may structurally stabilize the ultrasound transducer elements. This may also make the step of cutting the ultrasound transducer elements simpler.

In some embodiments, the method further comprising,
providing a common ground electrode, and
arranging the common ground electrode above the plurality of ultrasound transducer elements and subsequently arranging the first acoustic matching layer above the common ground electrode.

Consequently, the ultrasound transducer may be shaped curvilinear with the common ground electrode arranged above the plurality of ultrasound transducer elements.

A layer of adhesive may be provided between the ultrasound transducer elements and the common ground electrode and / or layer of adhesive may be provided between common ground electrode and the first acoustic matching layer. The common ground electrode may be a copper ground electrode.

In some embodiments, the first acoustic matching layer is arranged above the piezoelectric block before the piezoelectric block has been cut.

Consequently, transfer of acoustic energy between transducer elements may be limited.

In some embodiments, the method further comprising,
providing a common ground electrode, and
arranging the common ground electrode above the piezoelectric block and subsequently arranging the first acoustic matching layer above the common ground electrode.

The common ground electrode may be arranged on top side of the upper side of the piezoelectric block. An adhesive may be provided between the common ground electrode and the upper side of the piezo electric block. The common ground electrode may have a width being wider than the width of the upper side of the piezoelectric block, whereby a subsequent cutting of the piezo electric block may be done while keeping all parts of the common ground electrode electrically connected.

In some embodiments, the method further comprising,
providing a flexible electrical circuit for electrically receiving and providing electrical signals to the plurality of ultrasound transducer elements wherein the flexible electrical circuit comprising a plurality of first electrical contacts each first electrical contact being connectable to an ultrasound transducer element for providing and receiving electrical signals to the ultrasound transducer element,
attaching the flexible electrical circuit to the lower side of the piezoelectric block before moving the support structure and pressure element together.

Consequently, by using a flexible electrical circuit the piezo electric block may be provided with electrical contacts for the ultrasound transducer elements before the piezo electric block is curvilinear shaped.

The flexible electrical circuit may be a flexible printed circuit. An adhesive may be provided between the lower side of the piezoelectric block and the flexible electrical circuit.

In some embodiments, the flexible electrical circuit is attached to the lower side of the piezoelectric block before the piezoelectric block has been cut, and wherein cutting of the piezoelectric block electrically insulates the plurality of first electrical contacts.

Before cutting, the plurality of first electrical contacts are electrically connected via the piezoelectric block. However, after cutting each first electrical contact is preferably connected to only a single transducer element.

Only the piezoelectric block may be cut, i.e. the flexible electrical circuit may be left substantially intact. This may allow the flexible electrical circuit to assist in stabilizing the plurality of ultrasound transducer elements before they are supported by the support structure.

In some embodiments, the method further comprising,
obtaining an acoustic lens, the acoustic lens being formed as a single element, wherein the acoustic lens has a lower surface having a concave curvature,
arranging the acoustic lens above the first acoustic matching layer.

Consequently, the acoustic lens may mechanically stabilize the curvilinear ultrasound transducer with the gas-filed gaps.

According to a second aspect, the present disclosure relates to a curvilinear ultrasound transducer for use in connection with an endoscope procedure, wherein the curvilinear ultrasound transducer comprises an ultrasound transducer array comprising a plurality of ultrasound transducer elements made of a piezoelectric material, a support structure having a curvilinear upper surface, and one or more acoustic matching layers, the ultrasound transducer array being arranged above the curvilinear upper surface of the support structure, the one or more acoustic matching layers being arranged above the ultrasound transducer array and wherein a gas-filled gap is provided between each of the plurality of ultrasound transducer elements resulting in a plurality of gas-filled gaps.

Consequently, by having gas-filled gaps between the ultrasound transducer elements, it may become easier to manufacture a curvilinear ultrasound transducer, as the piezoelectric material may be shaped curvilinear with layers such as an acoustic matching layer arranged on top.

In some embodiments, the ultrasound transducer further comprises an acoustic lens, the acoustic lens being formed as a single element and being arranged above the first acoustic matching layer.

Consequently, the acoustic lens may mechanically stabilize the curvilinear ultrasound transducer with the gas-filed gaps.

In some embodiments, each ultrasound transducer element of the plurality of ultrasounds transducer elements is provided with an individual acoustic matching layer.

Consequently, transfer of acoustic energy between transducer elements may be limited.

In some embodiments, the curvilinear ultrasound transducer further comprises a common acoustic matching layer arranged above the ultrasound transducer array.

Consequently, the common acoustic matching layer may mechanically stabilize the curvilinear ultrasound transducer with the gas-filed gaps.

In some embodiments, the curvilinear ultrasound transducer further comprises a flexible electrical circuit comprising a plurality of first electrical contacts each first electrical contact being connected to an ultrasound transducer element for providing and receiving electrical signals to the ultrasound transducer element, the flexible electrical circuit being arranged between the curvilinear upper surface of the support structure and the plurality of ultrasound transducer elements.

According to a third aspect, the present disclosure relates to an endoscope comprising an image capturing device and a curvilinear ultrasound transducer as disclosed in relation to the second aspect of the invention.

According to a fourth aspect, the present disclosure relates to a curvilinear ultrasound transducer manufactured according to a method as disclosed in relation to the first aspect of the invention.

The different aspects of the present disclosure can be implemented in different ways including methods of manufacturing a curvilinear ultrasound transducer, a curvilinear ultrasound transducer and an endoscope comprising a curvilinear ultrasound transducer described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows a method of manufacturing a curvilinear ultrasound transducer according to an embodiment of the disclosure.
Fig. 2 shows a method of manufacturing a curvilinear ultrasound transducer according to an embodiment of the disclosure.
Fig. 3 shows a method of manufacturing a curvilinear ultrasound transducer according to an embodiment of the disclosure.
Fig. 4 shows a method of manufacturing a curvilinear ultrasound transducer according to an embodiment of the disclosure.
Figs. 5 shows schematically an endoscope according to an embodiment of the disclosure.

### Detailed description

In the following description, reference is made to the accompanying figures, which show by way of illustration how the embodiments of the present disclosure may be practiced.

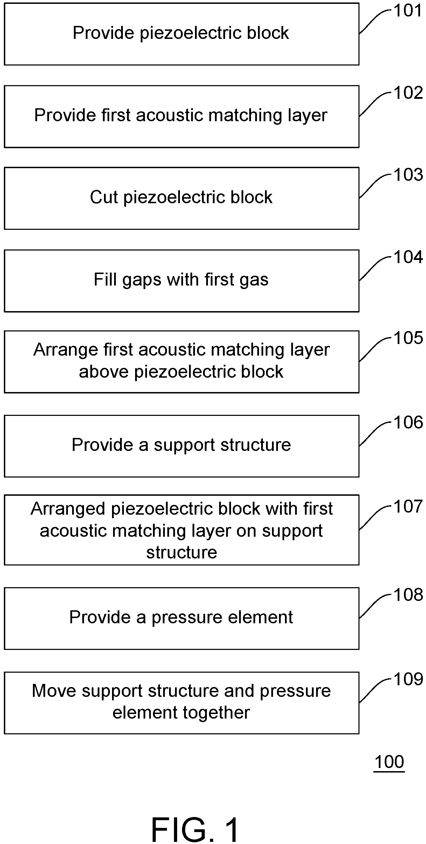

Fig. 1 shows a method 100 of manufacturing a curvilinear ultrasound transducer for use with an endoscope according to an embodiment of the disclosure. In step 101 a piezoelectric block having an upper side and a lower side opposite to the upper side is provided. In step 102, a first acoustic matching layer is provided. In step 103, the piezoelectric block is cut at a plurality of predetermined positions forming a plurality of ultrasound transducer elements separated by a plurality of gaps. In step 104, the plurality of gaps are allowed to be filed with a first gas resulting in a plurality of gas-filled gaps. In step 105, the first acoustic matching layer is arranged above the piezoelectric block, optionally after the piezoelectric block has been cut. In step 106, a support structure is provided having a curvilinear upper surface. In step 107, the piezoelectric block with the first acoustic matching layer is arranged above the curvilinear upper surface of the support structure, optionally after the piezoelectric block has been cut. In step 108, a pressure element is provided having a concave surface. In step 109, the support structure and the pressure element is moved together to shape the piezoelectric block curvilinear.

While the different steps of the method are shown in a particular order, it should be understood that the steps may be performed in a number of different orders. As an example, the cutting step 103 may be performed after the first acoustic matching layer is arranged above the piezoelectric block in step 105 but before the piezoelectric block has been shaped curvilinear in step 109. The cutting step 103 may also be performed at the end, i.e. after the piezoelectric block has been shaped curvilinear in step 109.

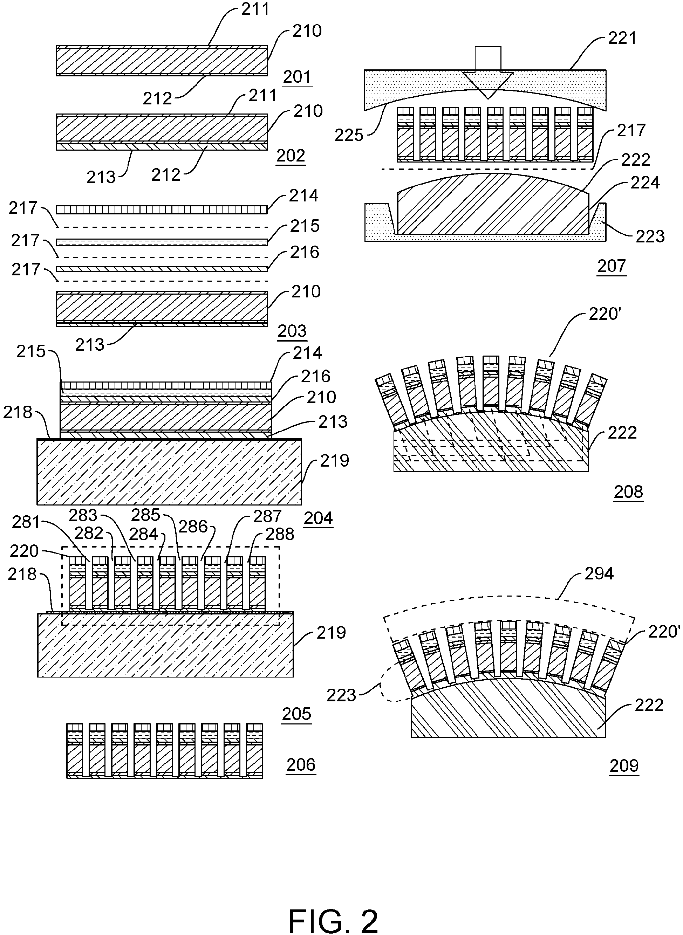

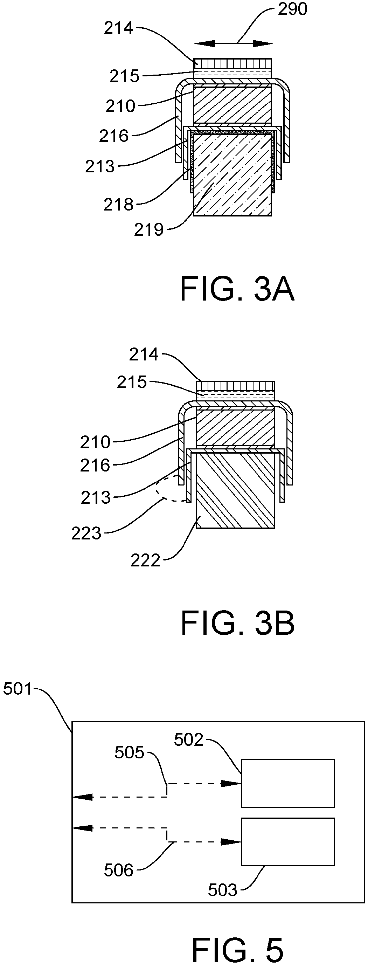

Fig. 2 shows a method of manufacturing a curvilinear ultrasound transducer for use with an endoscope according to an embodiment of the disclosure. Fig. 2 shows central cross-sections. In step 201 a piezoelectric block 210 is provided having an upper side and a lower side opposite to the upper side. A metal electrode 211 is provided on the upper side and a metal electrode 212 is provided on the lower side of the piezoelectric block 210. The piezoelectric block 210 may previously have been pre-poled and metalized. As an example, one or two layers of a nickel-chromium alloy may be applied to the piezoelectric block. Alternatively / additionally one or two layers of Au may be applied. The layer may preferably be thin layers having a thickness of 10-20nm. The layers may improve the piezoelectric properties of the piezoelectric block. Next in step 202, a flexible electrical circuit 213 is provided for electrically receiving and providing electrical signals to a plurality of ultrasound transducer elements. The flexible electrical circuit 213 comprises a plurality of first electrical contacts, where each first electrical contact is connectable to an ultrasound transducer element for providing and receiving electrical signals to the ultrasound transducer element. The flexible electrical circuit 213 is attached to the lower side of the piezoelectric block 210. The flexible electrical circuit 213 may be glued onto the lower side of the piezoelectric block 210. Next in step 203, a common ground electrode 216 is provided and arranged above the piezoelectric block 210. A layer of adhesive 217 is provided between the common ground electrode 216 and the piezoelectric block 210. Additionally, a first acoustic matching layer 215 and a second acoustic matching layer 214 is provided. The first acoustic matching layer 215 is arranged above the piezoelectric block 210 and attached to the common ground electrode 216 using an adhesive 217. The second acoustic matching layer 214 is arranged above the piezoelectric block 210 and attached to the first acoustic matching layer 215 using an adhesive 217. Then in step 204, the piezoelectric block 210 is arranged on a temporary support 219 to prepare the piezoelectric block 210 for cutting. The piezoelectric block 210 may be attached to the temporary support 219 using a release tape 218. The release tape may be a thermal release tape configured to adhere tightly to the piezoelectric block at a first temperature such as room temperature and release the piezoelectric block 210 at a second temperature such as a slightly elevated temperature. Alternatively, the release tape may be a UV release tape configured to adhere tightly to the piezoelectric block 210 before being exposed to UV radiation and release the piezoelectric block 210 after having been expose to UV radiation. If UV release tape is being used, the temporary support 219 is preferably made of a material permeable to UV radiation e.g. the temporary support 219 may be made of a glass material permeable to UV radiation. Next in step 205, the piezoelectric block 210 is cut at a plurality of predetermined positions thereby forming a plurality of ultrasound transducer elements 220 separated by a plurality of gaps. Fig. 3a shows a side view of the assembly in the state of step 205, i.e. after cutting. Preferably, the cutting / dicing blade may have a width corresponding the width of the plurality of gaps. The gaps are also known as the kerf of the ultrasound transducer. In the shown embodiment, 9 ultrasound transducer element are shown. However, typically more ultrasound transducer elements are formed e.g. at least 16, at least 32 or at least 64 ultrasound transducer elements. The cutting is done through the first acoustic matching layer 215, the second acoustic matching layer 214, (parts of) the common ground electrode 216 and the piezoelectric block 210. Preferably, the cutting is done through the entire piezoelectric block 210 but without significantly cutting into flexible electrical circuit 213. The common ground electrode 216 has a width being wider than the width 290 of the upper side of the piezoelectric block (as seen in Fig. 3a), whereby the cutting of the piezoelectric block can be done while keeping all parts of the common ground electrode 216 electrically connected. After cutting the plurality of gaps is allowed to be filed with a first gas resulting in a plurality of gas-filled gaps 281-288. Then, in step 206 the plurality of ultrasound transducer elements 220 are released from the release tape 218 and the temporary support 219. Next in step 207, a support structure 224 having a curvilinear upper surface 222 is provided. The piezoelectric block with the first acoustic matching layer 215, the second acoustic matching layer 214, and the common ground electrode 216 is arranged above the curvilinear upper surface of the support structure 222 with a layer of adhesive 217 provided between the piezoelectric block and the support structure 222. In this embodiment, this is done after the piezoelectric block has been cut. Additionally, a pressure element 221 is provided having a concave surface 225, and a support 223 for supporting the support structure 224 in step 207. Then the support structure 224 and the pressure element 221 is moved together to shape the piezoelectric block curvilinear 220' as shown in step 208. Next, in step 209 an electrical connection 223 is provided between common ground electrode 216 and the flexible electrical circuit 213. Fig. 3b shows a side view of the assembly in step 209. Finally, an acoustic lens 294 may be arranged above the first and second acoustic matching layer 215 216.

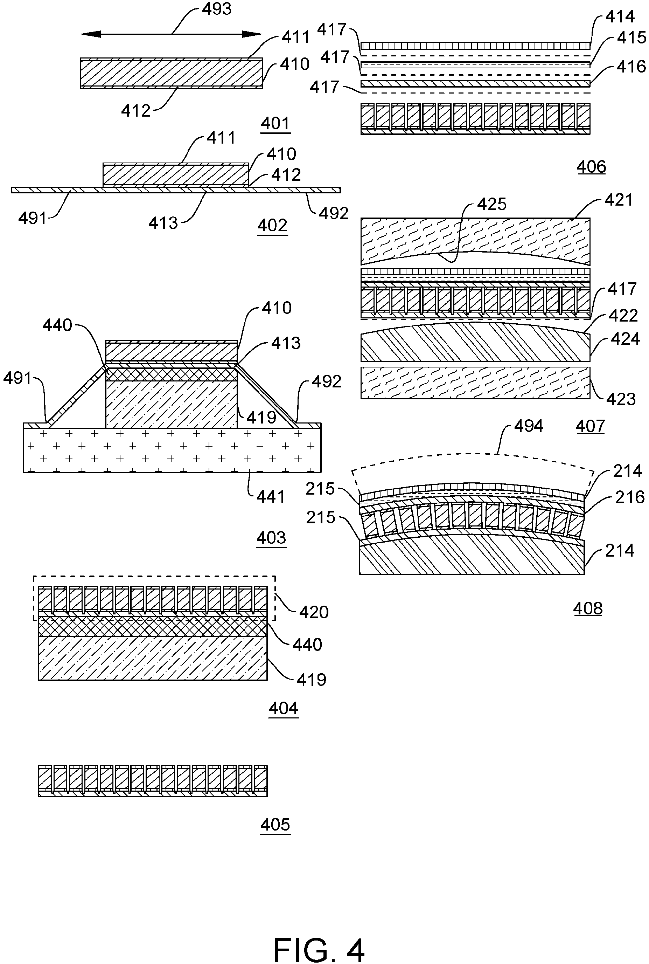

Fig. 4 shows a method of manufacturing a curvilinear ultrasound transducer for use with an endoscope according to an embodiment of the disclosure. Fig. 4 shows central cross-sections. In step 401 a piezoelectric block 410 is provided having an upper side and a lower side opposite to the upper side. A metal electrode 411 is provided on the upper side and a metal electrode 412 is provided on the lower side of the piezoelectric block 410. The piezoelectric block 410 may previously have been pre-poled and metalized. Next in step 402, a flexible electrical circuit 413 is provided for electrically receiving and providing electrical signals to a plurality of ultrasound transducer elements. The flexible electrical circuit 413 comprises a plurality of first electrical contacts, where each first electrical contact is connectable to an ultrasound transducer element for providing and receiving electrical signals to the ultrasound transducer element. The flexible electrical circuit 413 is attached to the lower side of the piezoelectric block 410. The flexible electrical circuit 413 may be attached using an adhesive (not shown) to the lower side of the piezoelectric block 410. In this embodiment, the flexible electrical circuit 413 has a first part 491 that extends beyond the length 493 of the piezoelectric block 410 and a second part 492 that extends beyond the length 493 of the piezoelectric block 410. The first part 491 and the second part 492 may be utilized for transferring the signals to and from the plurality of ultrasound transducer elements. Then in step 404, the piezoelectric block 410 is arranged on a temporary support 419 to prepare the piezoelectric block 410 for cutting. The piezoelectric block 410 is attached to the temporary support 419 using a release tape 240. The release tape may be a thermal release tape or a UV release tape. If UV release tape is being used, the temporary support 219 is preferably made of a material permeable to UV radiation e.g. the temporary support 219 may be made of a glass material permeable to UV radiation. Additionally, the first part of the flexible electric circuit 491 and the second part of the flexible electrical circuit 492 are attached to a support using a release tape such as a UV release tape or a thermal release tape. This may secure that the flexible electrical circuit 413 is stabilized during the cutting step and further protect the first part of the flexible electric circuit 491 and the second part of the flexible electrical circuit 492. Next in step 404, the piezoelectric block 410 is cut at a plurality of predetermined positions thereby forming a plurality of ultrasound transducer elements 420 separated by a plurality of gaps. From step 404, the first part and second part of the flexible electrical circuit 491 492 are no longer shown in fig. 4, however it should be understood that they are still present. Preferably, the cutting / dicing blade may have a width corresponding the width of the plurality of gaps. Preferably, the cutting is done through the entire piezoelectric block 410 but without significantly cutting into flexible electrical circuit 413. After cutting the plurality of gaps is allowed to be filed with a first gas resulting in a plurality of gas-filled gaps. Then in step 405, the plurality of ultrasound transducer elements 420 are released from the release tape 418 and the temporary support 419 and the first part and second part of the flexible electrical circuit 491 492 are released from the release tape 441. Then in step 406, a common ground electrode 416 is provided and arranged above the plurality of cut ultrasound transducer elements 420. A layer of adhesive 217 is provided between the common ground electrode 213 and the piezoelectric block 210. Additionally, a first acoustic matching layer 415 and a second acoustic matching layer 414 is provided. The first acoustic matching layer 415 is arranged above the plurality of cut ultrasound transducer elements 420 and attached to the common ground electrode 416 using an adhesive 417. The second acoustic matching layer 414 is arranged above the plurality of cut ultrasound transducer elements 420 and attached to the first acoustic matching layer 415 using an adhesive 417. In this embodiment, the acoustic matching layers 415 416 are arranged above the plurality of ultrasound transducer elements 420 after the piezoelectric block 410 has been cut forming the plurality of ultrasound transducer elements 420. Consequently, the acoustic matching layers 415 414 may structurally stabilize the ultrasound transducer elements. This may also make the step of cutting the ultrasound transducer elements 420 simpler.

Next in step 407, a support structure 424 having a curvilinear upper surface 422 is provided. The piezoelectric block with the first acoustic matching layer 415, the second acoustic matching layer 414, and the common ground electrode 416 is arranged above the curvilinear upper surface of the support structure 422 with a layer of adhesive 417 provided between the piezoelectric block and the support structure 422. In this embodiment, this is done after the piezoelectric block has been cut. Additionally, a pressure element 421 is provided having a concave surface 425, and a support 423 for supporting the support structure 424 in step 407. Then the support structure 424 and the pressure element 421 are moved together to shape the piezoelectric block curvilinear as shown in step 408. Finally, an acoustic lens 494 may be arranged above the first and second acoustic matching layer 415 416. By having the plurality of gaps filled with a first gas, it becomes simpler to manufacture a curvilinear ultrasound transducer, as the ultrasound transducer may be given the curvilinear shape with the acoustic matching layers and common ground electrode arranged on the piezoelectric block. This may reduce the number of process steps. Furthermore, the acoustic matching layers and common ground electrode may be manufactured with a planar shape, which may be significant simpler than attempting to match the specific curvilinear shape of the piezoelectric block. This may reduce the cost of manufacturing a curvilinear ultrasound transducer thereby extending the availability of endoscopic ultrasound. Furthermore, single use ultrasound endoscope may also become possible, which may eliminate risk of cross-contamination of serious illnesses.

Fig. 5 shows a schematic drawing of an endoscope 501 comprising an image capturing device 502 and a curvilinear ultrasound transducer 503. The curvilinear ultrasound transducer 503 may be a curvilinear ultrasound transducer as shown in 209 of Fig. 2 or step 408 of Fig. 4. The image capturing device 502 is configured to be communicatively coupled 405 to an image processing device (now shown) connected to a display or forming part of a display. Correspondingly, the curvilinear ultrasound transducer 503 is configured to be communicatively coupled 406 to an image processing device (now shown) connected to a display or forming part of a display.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A method of manufacturing a curvilinear ultrasound transducer for use with an endoscope, the method comprising,
providing a piezoelectric block having an upper side and a lower side opposite to the upper side,
providing a first acoustic matching layer,
cutting the piezoelectric block at a plurality of positions forming a plurality of ultrasound transducer elements separated by a plurality of gaps,
allowing the plurality of gaps to be filled with a first gas resulting in a plurality of gas-filled gaps,
arranging the first acoustic matching layer above the piezoelectric block, optionally after the piezoelectric block has been cut,
providing a support structure having a curvilinear upper surface,
arranging the piezoelectric block with the first acoustic matching layer above the curvilinear upper surface of the support structure, optionally after the piezoelectric block has been cut,
providing a pressure element, and
moving the support structure and the pressure element together to shape the piezoelectric block and first acoustic matching layer curvilinear.

2. A method of manufacturing a curvilinear ultrasound transducer according to claim 1, wherein the first gas is atmospheric air.

3. A method of manufacturing a curvilinear ultrasound transducer according to claims 1 or 2, wherein the first acoustic matching layer is arranged above the plurality of ultrasound transducer elements after the piezoelectric block has been cut forming the plurality of ultrasound transducer elements.

4. A method of manufacturing a curvilinear ultrasound transducer according to claim 3, wherein the method further comprising,
providing a common ground electrode, and
arranging the common ground electrode above the plurality of ultrasound transducer elements and subsequently arranging the first acoustic matching layer above the common ground electrode.

5. A method of manufacturing a curvilinear ultrasound transducer according to claims 1 or 2, wherein the first acoustic matching layer is arranged above the piezoelectric block before the piezoelectric block has been cut.

6. A method of manufacturing a curvilinear ultrasound transducer according to claim 5, wherein the method further comprising,
providing a common ground electrode, and
arranging the common ground electrode above the piezoelectric block and subsequently arranging the first acoustic matching layer above the common ground electrode.

7. A method of manufacturing a curvilinear ultrasound transducer according to any one of claims 1 to 6, wherein the method further comprising,
providing a flexible electrical circuit for electrically receiving and providing electrical signals to the plurality of ultrasound transducer elements wherein the flexible electrical circuit comprising a plurality of first electrical contacts each first electrical contact being connectable to an ultrasound transducer element for providing and receiving electrical signals to the ultrasound transducer element,
attaching the flexible electrical circuit to the lower side of the piezoelectric block before moving the support structure and pressure element together.

8. A method of manufacturing a curvilinear ultrasound transducer according to claim 7, wherein the flexible electrical circuit is attached to the lower side of the piezoelectric block before the piezoelectric block has been cut, and wherein cutting of the piezoelectric block electrically insulates the plurality of first electrical contacts.

9. A curvilinear ultrasound transducer for use in connection with an endoscope procedure, wherein the curvilinear ultrasound transducer comprises an ultrasound transducer array comprising a plurality of ultrasound transducer elements made of a piezoelectric material, a support structure having a curvilinear upper surface, and one or more acoustic matching layers, the ultrasound transducer array being arranged above the curvilinear upper surface of the support structure, the one or more acoustic matching layers being arranged above the ultrasound transducer array and wherein a gas-filled gap is provided between each of the plurality of ultrasound transducer elements resulting in a plurality of gas-filled gaps.

10. A curvilinear ultrasound transducer according to claim 9, wherein the ultrasound transducer further comprises an acoustic lens, the acoustic lens being formed as a single element and being arranged above the first acoustic matching layer.

11. A curvilinear ultrasound transducer according to claims 9 or 10, wherein each ultrasound transducer element of the plurality of ultrasounds transducer elements is provided with an individual acoustic matching layer.

12. A curvilinear ultrasound transducer according to claims 9 to 10, further comprising a common acoustic matching layer being arranged above the ultrasound transducer array.

13. A curvilinear ultrasound transducer according to any one of claims 9 to 12, further comprising a flexible electrical circuit comprising a plurality of first electrical contacts each first electrical contact being connected to an ultrasound transducer element for providing and receiving electrical signals to the ultrasound transducer element, the flexible electrical circuit being arranged between the curvilinear upper surface of the support structure and the plurality of ultrasound transducer elements.

14. An endoscope comprising an image capturing device and a curvilinear ultrasound transducer according to any one of claims 9 to 13.

15. A curvilinear ultrasound transducer manufactured according to any one of claims 1 to 8.
